(19) Europäisches Patentamt
European Patent Office
Office européen des brevets

(11) Veröffentlichungsnummer: **0 264 953 B1**

(12) # EUROPÄISCHE PATENTSCHRIFT

(45) Veröffentlichungstag der Patentschrift: **08.05.91**

(51) Int. Cl.5: **C07K 5/06,** A61K 37/02

(21) Anmeldenummer: **87115592.5**

(22) Anmeldetag: **23.10.87**

(54) **Verwendung von N, N'-bis-L-Aminosäure-L-cystinpeptiden in Aminosäure-Präparaten für orale und parenterale Ernährung.**

(30) Priorität: **24.10.86 DE 3636334**

(43) Veröffentlichungstag der Anmeldung:
**27.04.88 Patentblatt 88/17**

(45) Bekanntmachung des Hinweises auf die Patenterteilung:
**08.05.91 Patentblatt 91/19**

(84) Benannte Vertragsstaaten:
**AT BE CH DE ES FR GB GR IT LI NL SE**

(56) Entgegenhaltungen:
**EP-A- 0 087 750** **EP-A- 0 128 249**
**NL-A- 245 033** **US-A- 3 794 633**
**US-A- 3 875 136** **US-A- 4 284 624**

**JOURNAL OF ORGANIC CHEMISTRY, Band 32, Januar 1967, Seiten 97-102; R.G. HISKEY et al.: "Chemistry of aliphatic disulfides. XII. Synthesis of unsymmetrical open-chain cystine derivatives"**

(73) Patentinhaber: **PFRIMMER KABI GMBH & CO. KG**
**Hofmannstrasse 26**
**W-8520 Erlangen(DE)**

(72) Erfinder: **Stehle, Peter, Dr.**
**Universität Hohenheim Garbenstrasse 30**
**W-7000 Stuttgart 70(DE)**
Erfinder: **Fürst, Peter, Prof. Dr.**
**Universität Hohenheim Garbenstrasse 30**
**W-7000 Stuttgart 70(DE)**
Erfinder: **Fekl, Werner, Dr.Dr. h.c.**
**Altenseestrasse 19**
**W-8551 Röttenbach(DE)**

(74) Vertreter: **Freiherr von Pechmann, Eckehart et al**
**Wuesthoff & Wuesthoff Patent- und Rechtsanwälte Schweigerstrasse 2**
**W-8000 München 90(DE)**

EP 0 264 953 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

CHEMICAL ABSTRACTS, Band 86, 1977, Seite 555, Zusammenfassung Nr. 107036u, Columbus, Ohio, US; A. GOSDEN et al.: "Amino-acids and peptides. Part XL. Protection removable by electrolytic reduction: the use of S-4-picolyl-L-cysteine and O-4-picolyl-L-tyrosine in synthesis", & J. CHEM. RES., PART S (SYNOP.) 1977, (1), 22-3

CHEMICAL ABSTRACTS, Band 85, 1976, Seite 564, Zusammenfassung Nr. 71026d, Columbus, Ohio, US; W.C. STALLINGS, Jr. et al.: "A refinement of the crystal structure of N,N'-diglycyl-L-cystine dihydrate", & ACTA CRYSTALLOGR., SECT. B 1976, B32(6), 1916-17

CHEMICAL ABSTRACTS, Band 91, 1979, Seite 304, Zusammenfassung Nr. 170996a, Columbus, Ohio, US; M.D. ARMSTRONG: "Chromatographic properties of peptides of cystine and glycine and some related derivatives", & J. CHROMATOGR. 1979, 175(1), 216-18

## Beschreibung

Die schwefelhaltige Aminosäure Cystein und ihr duch Oxidation daraus leicht entstehendes stabiles dimeres Disulfid-Produkt Cystin gehören zwar nicht zu den klassischen essentiellen Aminosäuren, dennoch wird das Cystin bei der Ernährung von Säuglingen, bei chronischer Urämie, bei Leberzirrhose und bei Verletzungen als unentbehrlicher Bestandteil in den hierfür angebotenen Aminosäure-Präparaten eingestuft. Grund für die Essentialität in diesen Fällen ist eine ungenügende oder vollkommen fehlende Synthese von Cystin aus den als Vorstufen dienenden schwefelhaltigen Aminosäuren Methionin bzw. Cystein im Organismus.

Da in vielen Fällen die Neu- oder Frühgeborenen als auch die Patienten mit den erwähnten Krankheitsbildern intravenös ernährt werden müssen, ist es notwendig, Cystin in adäquaten Mengen in die entsprechenden Nährlösungen einzubringen. Dies wird jedoch durch die sehr geringe Wasserlöslichkeit von freiem Cystin von nur 0,1 g je Liter $H_2O$ verhindert.

Durch Acetylierung von Cystein hat man bereits ein lösliches Derivat geschaffen, das in geringer Menge in Aminosäure-Präparaten Verwendung findet. Allerdings wird auch diese Cysteinverbindung in wässriger Lösung schnell zum Disulfidprodukt oxidiert. Im Gegensatz zum freien Cystin ist aber dieses N,N'-bis-Acetyl-cystin relativ gut wasserlöslich, so daß es in Lösung bleibt, weshalb man auch versucht hat, dieses diacetylierte Cystin in den betreffenden Infusionspräparaten zu verwenden. Neueste Studien belegen jedoch eindeutig, daß acetylierte Aminosäuren im Menschen nur sehr schlecht verwertet und nur in sehr geringem Maße zum Aufbau von körpereigenem Eiweiß eingesetzt werden können. Seit langem ist bereits das Diglycyl-cystin als lösliche Cystinverbindung bekannt, das als Substrat für Peptidaseuntersuchungen verwendet wurde, da die Spaltung dieses Peptides wegen der Ausfällung des unlöslichen Cystins leicht gemessen werden kann; vgl. J. Biol. Chemistry, Bd, 128, 241-243 (1939). In Chemical Abstracts, Bd. 85, 1976, S. 564, Nr. 71026d ist über die Kristallstruktur des N,N'-Diglycyl-L-cystin-dihydrates berichtet und in Chemical Abstracts, Bd. 86, 1977, S. 555, Nr. 107036u ist eine Synthese dieses Tripeptids mittels elektrolytischer Reduktion erwähnt. Für Ernährungszwecke wurde dieses Cystinpeptid in der Praxis aber bisher noch nicht verwendet, obwohl hierfür ein Bedürfnis besteht.

Bekannt ist auch, daß man schwer lösliche Aminosäuren, wie Tyrosin und auch Cystin, durch Kondensieren mit der zweibasischen Aminosäure Lysin in sehr gut wasserlösliche Peptide überführen kann. So ist u.a. in der EP-PS 87 751 die Herstellung und Verwendung des $N^2$L-Cystinyl-$N^6$L-cystinyl-L-lysins beschrieben, eines trotz der zwei Cystinylgruppen gut wasserlöslichen Tripeptids, bei dem die zwei Cystinylreste mit jeweils einer ihrer Carboxylgruppen an die beiden Aminogruppen des Lysins gebunden sind.

Versuche, durch Synthese eines Tripeptids aus 2 Mol Alanin und 1 Mol Cystin, bei denen in analoger Weise wie zu den vorbekannten Lysinderivaten jeweils die Aminogruppe der Alaninmoleküle mit den Carboxylgruppen des Cystins gebunden sind, ein einfacheres und billigeres, aber auch gut metabolisierbares wasserlösliches Cystintripeptid zu schaffen, haben zwar gezeigt, daß dieses L-Cystinyl-bis-L-alanin, bei dem die beiden Carboxylgruppen des Cystins jeweils mit der Aminogruppe des Alanins verbunden sind, gut wasserlöslich ist, jedoch bei der Hitzesterialisation (121 °C) instabil ist und bereits nach 8 Minuten zur Bildung anderer Derivate führt, wo-durch die Bioverfügbarkeit des Cystins vermindert ist bzw. verloren geht.

Überraschenderweise wurde nun festgestellt, daß im Gegensatz zu diesem Tripeptid das N,N'-bis-L-Alanyl-L-cystin-peptid bzw. generell die N,N'-bis-L-Aminosäure-L-cystin-peptide der allgemeinen Formel

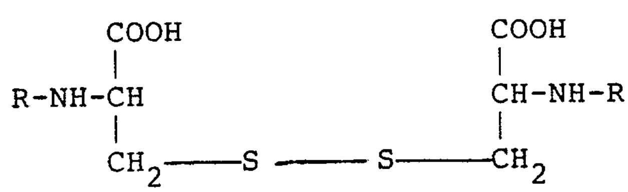

worin R einen L-Aminosäurerest bedeutet, nicht nur gut wasserlöslich sind, sondern auch bei der Hitzesterilisation in wässriger Lösung stabil bleiben, also hierbei nicht verändert worden sind und trotz dieser Stabilität im Organismus dann leicht gespalten und verwertet werden und daher gut bioverfügbare Cystinverbindungen darstellen. Erfindungsgemäß werden sie daher als Cystin-liefernde Verbindungen in Aminosäure- und Oligopeptid-Präparaten für die orale und parenterale Ernährung verwendet.

Da die Aminosäuren-Zusammensetzung bei den beiden Peptidarten, z.B. bei dem L-Cystinyl-bis-L-alanin und dem erfindungsgemäßen bis-L-Alanyl-L-cystin, identisch ist, muß die Erklärung der überraschenden Stabilität der einen Tris-Aminosäurepeptidart in der unterschiedlichen Aminosäuren-Sequenz liegen. Befindet sich der Cystin-Rest in N-terminaler Position, liegen die beiden freien Aminogruppen von Cystin räumlich sehr nahe beieinander. Dies könnte unter thermischer Belastung zu einer intramolekularen Cyclisierung unter Ammoniak-Abspaltung führen. Wird Cystin jedoch in die C-terminale Position eingebaut, befinden sich die freien Aminogruppen an den substituierenden Aminosäure-Resten. Eine Ringbildung ist unter diesen Bedingungen dann so gut wie ausgeschlossen, da die Aminogruppen räumlich voneinander getrennt sind. Eine Cyclisierung zwischen den beiden freien Carboxylgruppen (unter Abspaltung von $CO_2$) scheint aufgrund der geringen Nucleophilie dieser Gruppen bei der Erhitzung nicht einzutreten. Dies zeigt auch der Befund, daß alle bei der Hitzebehandlung des instabilen L-Cystinyl-bis-L-alanins gebildeten Sterilisationsprodukte bei 254 nm absorbieren. Das bedeutet, daß die Disulfidbindung der Cystingruppe während der Sterilisation nicht angegriffen wird. Zudem zeigten alle bei der thermischen Behandlung entstandenen neuen Produkte eine hohe UV-Absorption bei 206 nm, woraus gefolgert werden kann, daß die Peptidbindung als solche noch in unveränderter Form vorliegt.

Aus den vorliegenden Ergebnissen läßt sich schließen, daß Cystin-Tripeptide mit Carboxyl-ständigem Cystin-Rest unabhängig von der Art der N-ständigen Aminosäure eine bei der Sterilisation der Lösungen für die parenterale Ernährung ausreichende thermische Stabilität besitzen. Dies gilt nicht nur für das N,N'-bis-Alanyl-tripeptid des Cystins, sondern auch für analoge Cystin-peptide, wie das an sich seit längerem bekannte N,N'-bis-Glycyl-L-cystin das bisher nicht für die angegebene Verwendung eingesetzt worden ist sowie auch die bisher nicht bekannten Peptide N,N'-bis-L-Leucyl-L-cystin, N,N'-bis-L-Isoleucyl-L-cystin oder N,N'-bis-L-Phenylalanyl-L-cystin, die daher erfindungsgemäß geeignet sind für die Verwendung als Cystinderivate in Aminosäure- bzw. Oligopeptid-Präparaten für die orale oder parenterale Ernährung, wobei sich jedoch das N,N'-bis-L-Alanyl-L-cystin hierfür besonders gut eignet.

Die Diaminosäure-cystin-peptide lassen sich in üblicher Weise mittels der N-Carboxy-Anhydrid-Methode (NCA-Methode) herstellen, wobei dann die freie Aminogruppe von S-Acetamindomethyl-L-cystein mit dem jeweiligen N-Carboxyanhydrid der anzuhängenden Aminosäure reagiert, analog dem von R. Hirschmann et al in J. Org. Chem., Bd. 32, 3415-3425 (1967); J. Am. Chem. Soc., Bd. 93, 2746-2755 (1971) beschriebenen Verfahren. Durch anschließende Abspaltung der Schutzgruppe und Oxidation wird dann das entsprechende Cystin-Peptid gewonnen. Die neuen bis-Aminosäure-cystin-peptide, mit Ausnahme des bekannten N,N'-bis-Glycyl-cystins, werden erfindungsgemäß unter Anwendung dieser Verfahrensweise hergestellt.

Zu physiologischen Untersuchungen der in vivo-Verwertung von bis-Aminosäure-cystin-peptiden wurden bis-Glycyl-L-cystin und bis-L-Alanyl-L-cystin als Beispiele ausgewählt und männlichen Wistar-Ratten eine Peptid-Einmalinjektion (50 µmol/100 g KG bzw. 20 µmol/100 g KG, Injektionsvolumen 0.5 - 1 ml) über einen vena cava-Katheter innerhalb von 5 sec injiziert. Die Blutentnahme (1 ml in heparinisierten Spritzen) erfolgte 30, 60, 120, 180 und 300 sec nach Gabe des Peptids. Das durch Zentrifugation gewonnene Plasma wurde anschließend zur Proteinfällung mit 30% SSA (Verhältnis Probe zu SSA 10:1) versetzt, 1 h bei 4 °C gehalten und anschließend abzentrifugiert.

Zur Bestimmung der Peptide bis-Glycyl-L-cystin, mono-Glycyl-L-cystin, bis-L-Alanyl-L-cystin, mono-L-Alanyl-L-cystin sowie der freien Aminosäuren Glycin, Alanin und Cystin wurden die gefällten Plasma-Proben nach Vorsäulenderivatisierung mit 5-Dimethylaminonaphtalin-1-sulphonylchlorid-(DANSYL-C1) hochdruckflüssigchromatographisch analysiert.

Die analytischen Bedingungen sind im folgenden zusammengefaßt:

RP-HPLC nach DANSYL-Vorsäulenderivatisierung

| | |
|---|---|
| Säule: | Spherisorb ODS II 3 µm (125 x 4.6 mm) |
| Eluent: | (A) 12.5 mM Na-phosphat-Puffer pH 6.6 |
| | (B) 40% A; 60% ACN |
| Fluß: | 1.2 ml/min |
| Detektion: | Fluoreszenz Em 550/Ex 325 |

a) Injektion von Bis-Glycin-cystin = $(Gly\text{-}Cys)_2$

In Abbildung 1 sind die jeweils gemessenen Peptid/Aminosäure-Konzentrationen zu den verschiedenen Probenentnahmezeitpunkten nach Einmalinjektion von $(Gly\text{-}Cys)_2$ (50 µmol/100 g KG) dargestellt. Die Konzentration des Tripeptids $(Gly\text{-}Cys)_2$ nimmt innerhalb des Untersuchungszeitraumes kontinuierlich ab, wobei 5 min nach der Injektion nur noch ca. 10% des nach 30 sec gemessenen Wertes erreicht wird. Neben $(Gly\text{-}Cys)_2$ läßt sich in allen Proben das monosubstituierte Dipeptid $Gly\text{-}(Cys)_2$ (Zwischenprodukt bei der hydrolytischen Spaltung des Tripeptids $[Gly\text{-}Cys]_2$) nachweisen. Während die Konzentration dieses Dipeptids zu den ersten beiden Meßzeitpunkten konstant bleibt, fällt sie danach nahezu parallel zu $(Gly\text{-}Cys)_2$ ab.

Bereits 30 sec nach der Injektion sind die Konzentrationen von Glycin bzw. Cystin bereits um ein Vielfaches (Gly: 12fach; $(Cys)_2$: 14fach) gegenüber den Ausgangskonzentrationen erhöht. Obwohl die Konzentration von Glycin zu allen Zeitpunkten auf ähnlich hohem Niveau bleibt, läßt der Kurvenverlauf zwei Maxima bei 60 sec bzw. 180 sec erkennen. Diese Beobachtung könnte durch die schrittweise Hydrolyse von $(Gly\text{-}Cys)_2$ erklärt werden, bei der in der ersten Stufe Glycin und das monosubstituierte Dipeptid $Gly\text{-}(Cys)_2$freigesetzt und in der zweiten Phase das Dipeptid in die Aminosäuren Glycin und Cystin gespalten wird. Hierbei scheint es zunächst zu einer schnellen Spaltung des vorhandenen Tripeptids und erst nach Abnahme der Tripeptidkonzentration zur weiteren Hydrolyse des Dipeptids zu kommen, was durch die gemessenen Cystinwerte nachgewiesen ist. Im Gegensatz zu Glycin steigt die Cystinkonzentration bis 5 min nach der Injektion kontinuierlich an und erreicht einen Wert, der ca. 30fach über dem Ausgangswert liegt.

Wird zur kinetischen Auswertung ein 2-Kompartment-Modell zugrunde gelegt, läßt sich unter Anwendung der Residual-Methode eine Halbwertszeit von $240 \pm 89$ sec (n = 5) berechnen.

In Abbildung 2 sind die nach der Injektion von 20 µmol $(Gly\text{-}Cys)_2$/100 g KG ermittelten Plasmakonzentrationen zu den verschiedenen Entnahmezeitpunkten dargestellt. Grundsätzlich sind hier sehr ähnliche Konzentrationsverläufe zu beobachten. Zum ersten Meßzeitpunkt sind die Konzentrationen der beiden Aminosäuren Glycin und Cystin beträchtlich gegenüber den Ausgangspunkt erhöht (Gly: 5fach; $(Cys)_2$: 10fach).

Während die Konzentration an Cystin kontinuierlich zunimmt, bleibt Glycin während den ersten 180 sec relativ konstant und fällt nach 300 sec leicht ab. Obwohl der nach 180 sec gemessene Wert etwas höher liegt als nach 120 sec, kann hier nicht von einem ausgeprägten zweiten Glycinmaximum gesprochen werden.

Interessanterweise werden bereits 30 sec nach der Injektion für das Hydrolysezwischenprodukt $Gly\text{-}(Cys)_2$ ähnliche Konzentrationen wie für $(Gly\text{-}Cys)_2$ gemessen. Die Konzentration beider Peptide nimmt dann in weiteren Verlauf kontinuierlich im gleichen Ausmaß ab.

b) Injektion von Bis-Alanyl-cystin = $(Ala\text{-}Cys)_2$

In Abbildung 3 sind die jeweils gemessenen Peptid/Aminosäurekonzentrationen zu den verschiedenen Probenentnahmezeitpunkten nach Injektion von $(Ala\text{-}Cys)_2$ (20 µmol/100 g KG) dargestellt.

Das infundierte Tripeptid $(Ala\text{-}Cys)_2$ konnte nur in den ersten 2 min in allen 6 Versuchstieren nachgewiesen werden. Bereits 180 sec nach der Injektion war $(Ala\text{-}Cys)_2$ nur noch in einer Probe in geringer Konzentration (ca. 25 µmol/ml) nachweisbar. Zum letzten Entnahmezeitpunkt konnte in keinem der Tiere $(Ala\text{-}Cys)_2$ nachgewiesen werden.

Die Konzentration des Hydrolysezwischenproduktes $Ala\text{-}(Cys)_2$ lag bereits 30 sec nach der Injektion ca. 5fach höher als diejenige des Tripeptides $(Ala\text{-}Cys)_2$. Im weiteren Verlauf nahm die Konzentration von $Ala\text{-}(Cys)_2$ sehr rasch ab. Bei Beendigung des Versuchs nach 300 sec konnten nur noch in einem Versuchstier geringste Mengen an $Ala\text{-}(Cys)_2$ nachgewiesen werden. Während des gesamten Meßzeitraumes nahm die Konzentration von Alanin, die anfänglich nahezu 7fach über dem Ausgangswert lag, kontinuierlich ab. Nach 300 sec wurde dabei ein dem 3fachen Ausgangsniveau entsprechender Wert erreicht. Die maximale Cystinkonzentration (das 23 fache der Ausgangskonzentration) wurde nach 60 sec gemessen. Während des Untersuchungszeitraumes nahm die Konzentration dieser Aminosäure zwar langsam, aber stetig ab.

Aufgrund der immens schnellen Peptidelimination war es anhand der vorliegenden Daten nicht möglich, ähnlich wie bei $(Gly\text{-}Cys)_2$ eine Halbwertszeit zu berechnen. Aus den vorliegenden Daten läßt sich lediglich schließen, daß die Plasma-Halbwertszeit unter 2 min liegen muß.

Die mit diesen beiden erfindungsgemäß zu verwendenden bis-Aminosäure-cystinpeptiden erhaltenen Ergebnisse zeigen eindeutig, daß sowohl $(Gly\text{-}Cys)_2$ als auch $(Ala\text{-}Cys)_2$ nach der Einmalinjektion rasch aus

dem Plasma geklärt werden. Das Auftreten des jeweiligen monosubstituierten Dipeptids und der schnelle Konzentrationsanstieg der entsprechenden freien Aminosäuren Cystin und Glycin bzw. Alanin im Verlgiech zu den Ausgangswerten lassen den Schluß zu, daß die Elimination der Peptide überwiegend auf einer schrittweisen hydrolytischen Spaltung von $(Gly\text{-}Cys)_2$ bzw. $(Ala\text{-}Cys)_2$ beruht. Die hohen Plasmakonzentrationen der monosubstituierten Dipeptide können hierbei als Hinweis für eine Hydrolyse im Plasma bzw. an der Plasmamembran interpretiert werden.

Wie aus den Abbildungen 2 bzw. 3 ersichtlich, wird das Tripeptid $(Ala\text{-}Cys)_2$ beträchtlich schneller in die freien Aminosäuren gespalten als das $(Gly\text{-}Cys)_2$. Während 30 sec. nach der Injektion von $(Gly\text{-}Cys)_2$ - (20 µmol/100 KG) noch 655 µmol/ml an Tripeptid nachgewiesen wurden, konnten nach entsprechender Einmalgabe von $(Ala\text{-}Cys)_2$ nur 280 µmol/ml Plasma bestimmt werden. Obwohl aufgrund der schnellen Hydrolyse von $(Ala\text{-}Cys)_2$ keine pharmakokinetische Auswertung möglich ist, ist es offensichtlich, daß die Halbwertszeit von $(Ala\text{-}Cys)_2$ beträchtlich kürzer ist als für $(Gly\text{-}Cys)_2$.

Die raschere in vivo-Nutzung von $(Ala\text{-}Cys)_2$ spiegelt sich auch in den Aminosäurenkonzentrationen wider. Während nach $(Gly\text{-}Cys)_2$-Gabe die Konzentration von Glycin innerhalb des Untersuchungszeitraumes auf gleichmäßigem Niveau bleibt und Cystin kontinuierlich ansteigt (Abb. 3), fällt die Konzentration von Alanin bzw. Cystin nach Injektion von $(Ala\text{-}Cys)_2$ bis zum letzten Entnahmezeitpunkt deutlicher ab, wobei die Maxima bereits nach 30 sec (Alanin) bzw. 60 sec (Cystin) beobachtet wurden.

Diese in vivo-Versuche zur Verwertbarkeit der erfindungsgemäß intravenös verabreichten cystinhaltigen Tripeptide liefern klare Beweise für eine rasche Aufnahme und anschließende Hydrolyse der Peptide $(Gly\text{-}Cys)_2$ und $(Ala\text{-}Cys)_2$.

Da die vorliegenden Ergebnisse darauf schließen lassen, daß die Peptide entweder im Plasma oder an der Plasmamembran gespalten werden, stellen die beträchtlich schnellere Nutzung von $(Ala\text{-}Cys)_2$ und die damit verbundene beschleunigte Elimination aus dem Plasma durch Aufnahme in die Zellen einen Vorteil des Alanin-Peptids im Vergleich zum $(Gly\text{-}Cys)_2$ dar. Obwohl beide Tripeptide in vivo verwertet und somit geeignete Cystinsubstrate für die parenterale und orale Ernährung darstellen, ist das bis-L-Alanyl-L-cystinpeptid als bevorzugte Cystinquelle anzusehen. Aber auch die höheren bis-Aminosäure-cystin-peptide, wie z.B. das bis-L-Leucyl-L-cystin-peptid, das bis-L-Isoleucyl-L-cystin-peptid oder das bis-L-Phenylalanyl-L-cystinpeptid eignen sich ebenfalls gut zur schnellen Verfügungsstellung von Cystin beim körpereigenen Eiweißaufbau, wobei im Falle der Verwendung essentieller Aminosäuren für die Cystinpeptide diese dem Körper zuzuführenden Aminosäuren mit dem Cystin dem Organismus zur Verfügung gestellt werden können.

Die N,N'-bis-L-Aminosäure-L-cystin-peptide der allgemeinen Formel

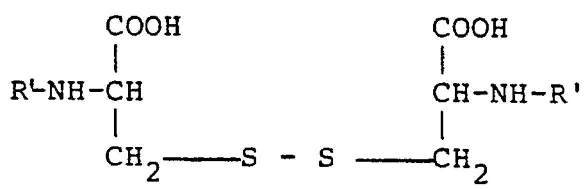

worin R' einen Aminosäure-Rest mit 3 oder mehr Kohlenstoffatomen bedeutet, sind neue Verbindungen und daher ein Gegenstand der Erfindung. Wie schon erwähnt können sie durch die N-Carboxy-Anhydrid-Methode aus Cystein und anschließender Oxidation hergestellt werden. Sie sind hitzestabil und werden dennoch im Organismus bzw. in den Zellen durch die betreffenden Peptidasen schnell gespalten, so daß das zum Aufbau körpereigenen Eiweißes wichtige Cystin in ausreichender Menge zur Verfügung steht.

Beispiel 1

Synthese von N,N'-bis-L-Alanyl-L-cystin mittels NCA-Methode

4 mmol (0,91 g) S-Acetamidomethyl-L-cystein (S-Acm-Cys) werden mit 4,5 mmol (0,52 g) Alanin-NCA in wäßriger Lösung (250 $cm^3$) bei pH 10,2 und 0 °C umgesetzt. Mittels Gel-Chromatographie wird das erhaltene Ala-S(Acm)Cys isoliert und dann die AMC-Schutzgruppe mit $Hg(CH_3COO)_2$ bei pH 4,0 abgespalten. Nach Fällung des Hg-Salzes mit $H_2S$ und anschließendem Abfiltrieren bei pH 7 wird zum Cystintripeptid oxidiert. Durch gel-chromatographische Aufarbeitung des oxidierten Ansatzes wird das N,N'-bis-L-Alanyl-

L-cystin mit Smp (Zersetzungspunkt: 195 - 200° C) isoliert.

Beispiel 2

Synthese von N,N'-bis-L-Isoleucyl-L-cystin mittels Aktivester-Methode

400 µmol (91,5 mg) S-Acetamidomethyl-L-Cystein . HCl werden in 4 ml 0,1 KCl gelöst, in ein thermostatisiertes Reaktionsgefäß (40° C) eingebracht und mit NaOH auf pH 9,2 eingestellt. Die Reaktion wird durch Zugabe von 200 µmol (72,5 g) Carbobenzoxy-L-Isoleucin-hydroxysuccinimidester, der in Dimethylsulfoxyd aufgelöst worden war, gestartet. Durch kontinuierliches Zutitrieren von NaOH wird der pH konstant gehalten. Nach Beendigung der Reaktion (4 h) wird der Ansatz zur Isolierung des Peptids gelchromatographisch aufgearbeitet. Danach erfolgt die Abspaltung der Carbobenzoxy-Schutzgruppe mittels katalytischer Hydrierung in an sich bekannter Weise in einem wäßrig-organischem Lösungsmittelgemisch in Gegenwart von Palladiumaktivkohle als Katalysator. Nach Abtrennen des Lösungsmittels wird die Acetamido-Schutzgruppe, wie im Beispiel 1 beschrieben, abgespalten und anschließend zum Cystintripeptid oxidiert. Nach gelchromatographischer Reinigung wird das N,N' bis-L-Isoleucyl-L-cystin mit Smp (Zersetzungspunkt: 209 - 213° C) isoliert.

Beispiel 3

Synthese von N,N'-bis-L-Phenylalanyl-L-cystin mittels der Aktivester-Methode

Es werden 400 µmol(91,5 mg) Acetamidomethyl-L-cysteins-HCl, wie in Beispiel 2 beschrieben, mit 200 µmol (79,3 mg) Carbobenzoxy-L-Phenylalanin-hydroxysuccinimidester umgesetzt. Nach Aufarbeitung und Abspaltung der Schutzgruppen und Oxidation (wie in Beispiel 2 beschrieben) wird das N, N'-bis-L-Phenylalanyl-cystin mit Smp (Zersetzungspunkt: 217 - 220° C) erhalten.

Sterilisationsversuche

Ausgehend von einer Stammlösung des erhaltenen erfindungsgemäßen Tripeptids (2 mmol/l in Tris-Puffer) wurden Lösungen mit den pH-Werten 5,5, 6,5, 7,5 und 8,5 durch Zugabe von 0,1 N HCL bzw. KOH hergestellt. Danach wurden jeweils 0,5 ml dieser Lösung in Chromschwefelsäure-gereinigte Ampullen gefüllt, unter Vakuum zugeschmolzen und 8, 12, 15 bzw. 30 Minuten bei 121° C sterilisert.

In analoger Weise wurden auch entsprechende Lösungen des Cystinyl-bis-alanin-tripeptids hergestellt und behandelt.

Sowohl die Stammlösungen als auch die sterilisierten Proben wurden dann mittels Kapillar-isotachophorese und HPLC analysiert. Im Gegensatz zum L-Cystinyl-bis-alanin-tripeptid waren die erfindungsgemäßen N,N'-bis-L-Aminosäure-L-cystin-peptide auch nach 30 min langer Erhitzung praktisch unverändert vorhanden, was deren überraschende Stabilität bei der Hitzesterilisation aufzeigt. Diese Tripeptide sind daher nicht nur für orale Präparate, sondern insbesondere auch für die Herstellung parenteraler Infusionslösungen geeignet.

Die folgenden Beispiele zeigen Infusionslösungen für parenterale Applikationen.

B e i s p i e l   4

| Peptide und Aminosäuren | g/1000 mL |
|---|---|
| $(Ala-Cys)_2$ | 4,8 |
| Gly-Tyr[1)] | 6,6 |
| L-Histidin | 5,5 |
| L-Isoleucin | 7,0 |
| L-Leucin | 11,0 |
| L-Lysin-Monoacetat | 7,7 |
| L-Methionin | 9,0 |
| L-Phenylalanin | 6,0 |
| L-Threonin | 5,0 |
| L-Tryptophan | 2,5 |
| L-Valin | 8,0 |

1) Glycyl-L-tyrosin

Dieses Dipeptid enthält L-Tyrosin in leicht löslicher Form.

## Beispiel 5

| Peptide und Aminosäuren | g/1000 mL |
|---|---|
| Ala-Gln[1)] | 17,8 |
| L-Isoleucin | 2,1 |
| L-Leucin | 2,9 |
| L-Lysin-acetat | 4,37 |
| L-Methionin | 1,80 |
| L-Threonin | 2,70 |
| L-Tryptophan | 1,00 |
| L-Valin | 2,30 |
| L-Arginin | 7,00 |
| L-Histidin | 1,75 |
| L-Alanin | 0,7 |
| L-Glutaminsäure | 5,00 |
| Glycin | 6,4 |
| L-Prolin | 7,0 |
| L-Serin | 6,2 |
| $(Phe\text{-}Cys)_2$ | 4,0 |
| Gly-Tyr[2)] | 2,6 |
| | |
| Kaliumhydroxid | 1,96 |
| Natriumchlorid | 2,57 |
| Calciumglycero-phosphat-2-hydrat | 0,49 |
| Magnesiumchlorid | 0,61 |
| Natriumglycerophosphat | 5,51 |
| Kaliumchlorid | 0,37 |

1) L-Alanyl-L-glutamin
2) Glycyl-L-tyrosin
Die beiden Dipeptide enthalten L-Glutamin bzw. L-Tyrosin in stabiler bzw. in leicht löslicher Form

Beispiel 6

| Peptide und Aminosäuren | g/1000 mL |
|---|---|
| Ala-Gln[1)] | 1,5 |
| L-Isoleucin | 7,60 |
| L-Leucin | 8,50 |
| L-Lysin-Monomalat | 7,67 |
| L-Methionin | 0,25 |
| L-Phenylalanin | 0,10 |
| L-Threonin | 1,20 |
| L-Tryptophan | 0,10 |
| L-Valin | 6,40 |
| L-Arginin | 4,90 |
| L-Histidin | 0,60 |
| L-Ornithin-L-aspartat | 8,03 |
| L-Alanin | 1,50 |
| L-Glutaminsäure | 1,00 |
| Glycin | 0,50 |
| L-Prolin | 1,20 |
| L-Serin | 1,75 |
| $(Gly\text{-}Cys)_2$ | 0,20 |
| Tyr-Lys (Tyr)[2)] | 0,20 |
| Natriumglycerophosphat-5-hydrat | 4,52 |
| Magnesiumchlorid | 1,02 |
| Kaliumchlorid | 1,34 |

1) L-Alanyl-L-glutamin
2) $N^2$-L-Tyrosinyl-$N^6$-L-tyrosinyl-L-lysin
Diese beiden Peptide enthalten L-Glutamin bzw. L-Tyrosin in stabiler bzw. in leicht löslicher Form.

Beispiel 7

| Peptide und Aminosäuren | g/1000 mL |
|---|---|
| (Ala-Cys)$_2$ | 4,8 |
| Tyr-Lys (Tyr)[1)] | 6,1 |
| L-Isoleucin | 6,0 |
| L-Leucin | 9,4 |
| L-Lysin-Monoacetat | 4,23 |
| L-Methionin | 6,5 |
| L-Phenylalanin | 6,0 |
| L-Threonin | 4,5 |
| L-Tryptophan | 2,2 |
| L-Valin | 7,0 |
| L-Histidin | 4,5 |
| L-Alanin | 7,0 |
| L-Arginin | 5,0 |
| L-Glutaminsäure | 4,5 |
| Glycin | 2,0 |
| L-Serin | 2,5 |
| L-Prolin | 5,0 |
| Gly-Gln[2)] | 8,3 |

1) $N^2$-L-Tyrosinyl-$N^6$-L-tyrosinyl-L-lysin
2) Glycyl-L-glutamin
Diese beiden Peptide enthalten L-Tyrosin bzw. L-Glutamin in leicht löslicher bzw. in stabiler Form

## Ansprüche

1. Verwendung der N,N'-bis-L-Aminosäure-L-cystin-peptide der allgemeinen Formel

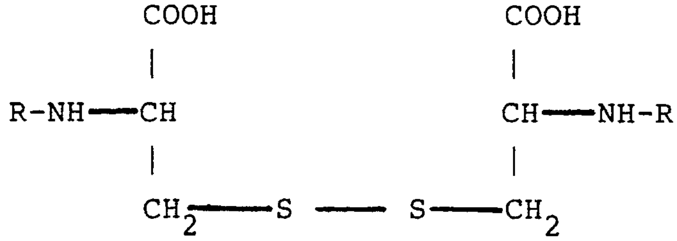

worin R einen L-Aminosäurerest bedeutet, als Cystin-liefernde Verbindung zur Herstellung eines

Aminosäure-Präparates für die orale oder parenterale Ernährung.

2. Verwendung von N,N'-bis-L-Glycin-L-cystin nach Anspruch 1.

3. Verwendung von N,N'-bis-L-Alanyl-L-cystin nach Anspruch 1.

4. Verwendung von N,N'-bis-L-Leucyl-L-cystin nach Anspruch 1.

5. Verwendung von N,N'-bis-L-Isoleucyl-L-cystin nach Anspruch 1.

Patentansprüche für folgende Vertragsstaaten: ES, GR

1. Verfahren zur Herstellung von Aminosäure-Präparaten zur Verwendung für die orale und parenteralen Ernährung, dadurch **gekennzeichnet**, daß man N,N'-bis-L-Aminosäure-L-cystinpeptid der allgemeinen Formel

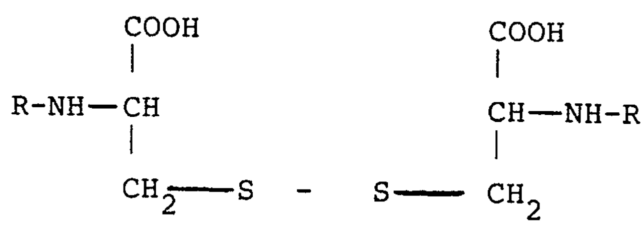

worin R einen L-Aminosäure-Rest bedeutet, als Cystin-liefernde Verbindung verwendet.

2. Verfahren nach Anspruch 1, dadurch **gekennzeichnet**, daß man das N,N'-bis-L-Glycyl-L-cystin verwendet.

3. Verfahren nach Anspruch 1, dadurch **gekennzeichnet**, daß man das N,N'-bis-L-Alanyl-L-cystin verwendet.

4. Verfahren nach Anspruch 1, dadurch **gekennzeichnet**, daß man das N,N'-bis-L-Leucyl-L-cystin verwendet.

5. Verfahren nach Anspruch 1,
dadurch **gekennzeichnet**,
daß man das N,N'-bis-L-Isoleucyl-L-cystin verwendet.

6. Verfahren zur Herstellung der N,N'-bis-L-Aminosäure-L-cystin-peptide mit Ausnahme des N,N'-bis-L-Glycyl-L-cystin-peptids für die Verwendung nach Anspruch 1 - 5,
dadurch **gekennzeichnet**,
daß man S-Acetamido-methyl-L-cystin mit dem jeweiligen N-Carboxy-anhydrid der anzuhängenden Aminosäure, mit Ausnahme des N-Carboxy-anhydrid des Glycins, umsetzt, die Schutzgruppen abspaltet und durch Oxidation das Cystin-tripeptid erhält.

## Claims

1. Use of N,N'-bis-L-amino acid-L-cystine peptides of the general formula

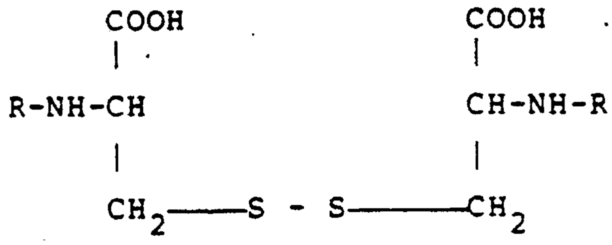

wherein R represents an L-amino acid residue as cystine providing compound for preparing an amino acid preparation for oral and parenteral nutrition.

2. Use of N,N'-bis-L-glycine-L-cystine according to claim 1.

3. Use of N,N'-bis-L-alanyl-L-cystine according to claim 1.

4. Use of N,N-bis-L-leucyl-L-cystine according to claim 1.

5. Use of N,N'-bis-L-isoleucyl-L-cystine according to claim 1.

Claims for the following Contracting States: ES, GR

1. A method of preparing amino acid preparations to be used for oral and parenteral nutrition, **characterized** in that N,N'-bis-L-amino acid L-cystine peptide of the general formula

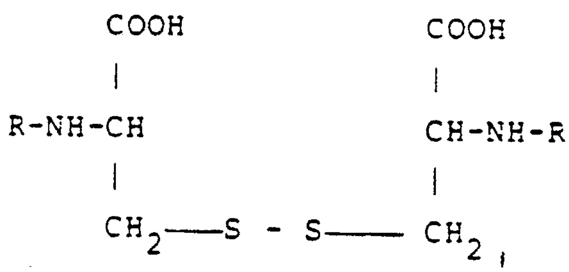

wherein R represents an L-amino acid residue, is used as cystine providing compound.

2. The method according to claim 1, **characterized** in that N,N'-bis-L-glycyl-L-cystine is used.

3. The method according to claim 1, **characterized** in that N,N'-bis-L-alanyl-L-cystine is used.

4. The method according to claim 1, **characterized** in that N,N'-bis-L-leucyl-L-cystine is used.

5. The method according to claim 1, **characterized** in that N,N'-bis-L-isoleucyl-L-cystine is used.

6. Method for preparing the N,N'-bis-L-amino acid-L-cystine peptides except for N,N'-bis-L-glycyl-L-cystine peptide for use according to claims 1 to 5,
**characterized** in that S-acetamidomethyl-L-cystine is reacted with the respective N-carboxyanhydride of the amino acid to be attached, except for the N-carboxy-anhydride of glycine, the protective groups are cleaved off, and cystine tripeptide is obtained by oxidation.

## Revendications

1. Utilisation des N,N'-bis-L-aminoacide-L-cystine-peptides de la formule générale:

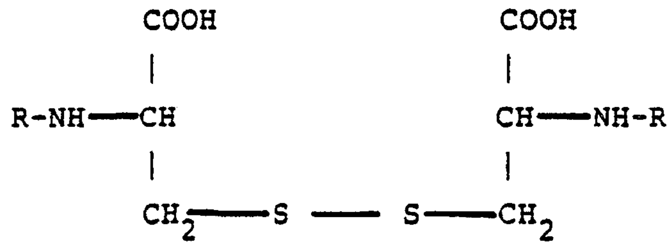

dans laquelle R représente un reste d'aminoacide, à titre de composé fournisseur de cystine pour la fabrication d'une préparation d'aminoacides destinée à l'alimentation par la voie orale ou parentérale.

2. Utilisation de la N,N'-bis-L-glycine-L-cystine selon la revendication 1.

3. Utilisation de la N,N'-bis-L-alanyl-L-cystine selon la revendication 1.

4. Utilisation de la N,N'-bis-L-leucyl-L-cystine selon la revendication 1.

5. Utilisation de la N,N'-bis-L-isoleucyl-L-cystine selon la revendication 1.

Revendications pour les Etats contractants suivants: ES, GR

1. Procédé de préparation de préparations d'aminoacides destinées à la nutrition par la voie orale ou parentérale, caractérisé en ce que l'on utilise des N,N'-bis-L-aminoacide-L-cystinepeptides de la formule générale:

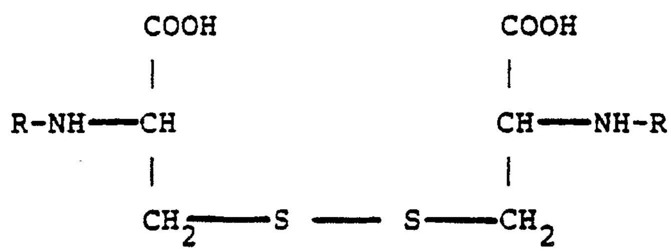

dans laquelle R représente le reste d'un aminoacide, à titre de composé fournisseur de cystine.

2. Procédé suivant la revendication 1, caractérisé en ce que l'on utilise la N,N'-bis-L-glycyl-L-cystine.

3. Procédé suivant la revendication 1, caractérisé en ce que l'on utilise la N,N'-bis-L-alanyl-L-cystine.

4. Procédé suivant la revendication 1, caractérisé en ce que l'on utilise la N,N'-bis-L-leucyl-L-cystine.

5. Procédé suivant la revendication 1, caractérisé en ce que l'on utilise la N,N'-bis-L-isoleucyl-L-cystine.

6. Procédé de préparation des N,N'-bis-L-aminoacide-L-cystinepeptides, à l'exception du N,N'-bis-L-glycyl-L-cystinepeptide, en vue de l'utilisation selon les revendications 1 à 5,
caractérisé en ce que l'on fait réagir la S-acétamido-méthyl-L-cystine avec le N-carboxy-anhydride concerné de l'aminoacide à accrocher, à l'exception du N-carboxy-anhydride de la glycine, on élimine les radicaux protecteurs et on obtient le cystine-tripeptide par oxydation.

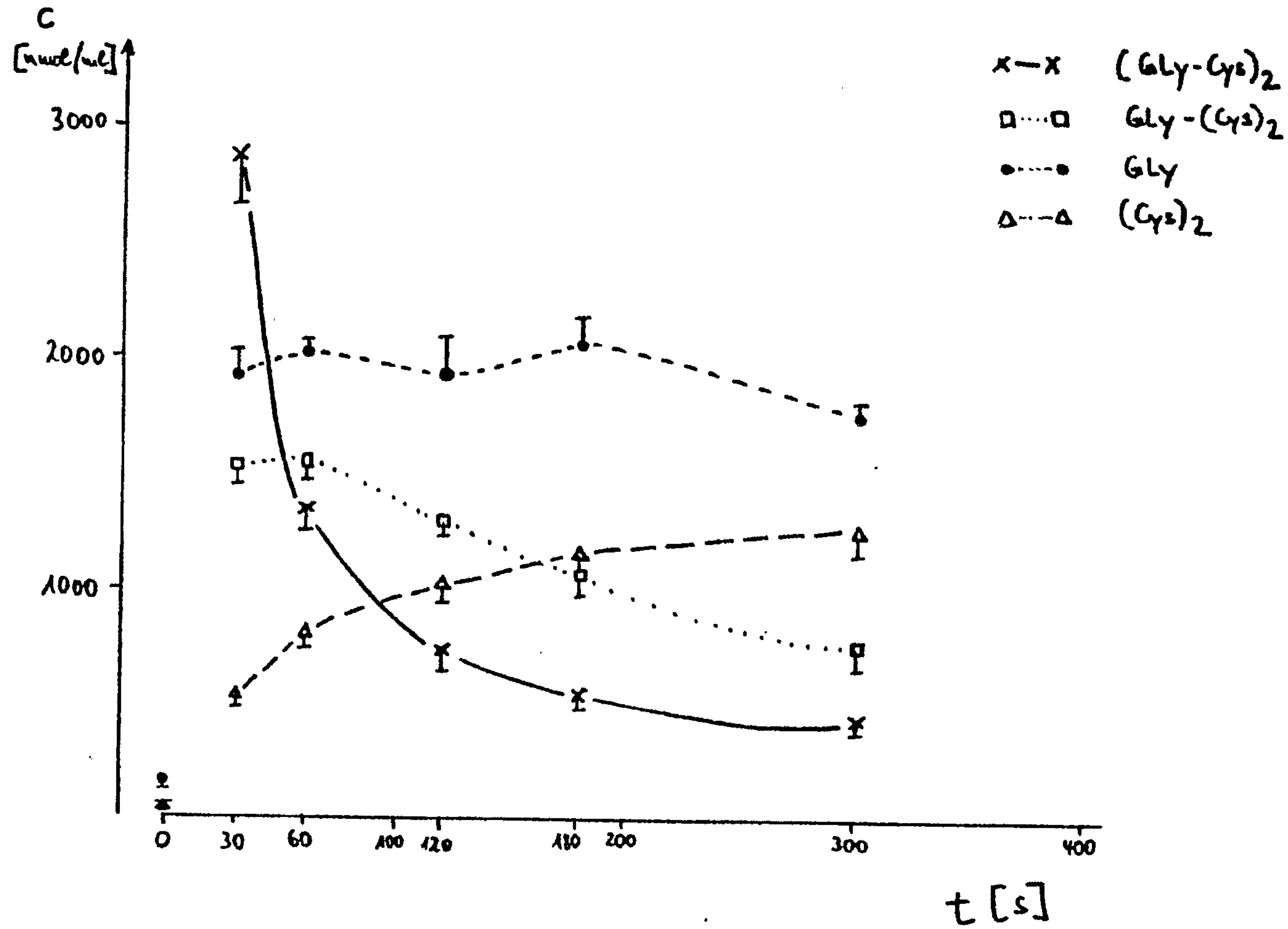


Abbildung 1: Plasma-Konzentrationen von $(Gly\text{-}Cys)_2$, $Gly\text{-}(Cys)_2$, Gly und $(Cys)_2$ nach Einmalinjektion von $(Gly\text{-}Cys)_2$ (50 µmol/100 g KG); Mittelwerte ± SEM, n = 6

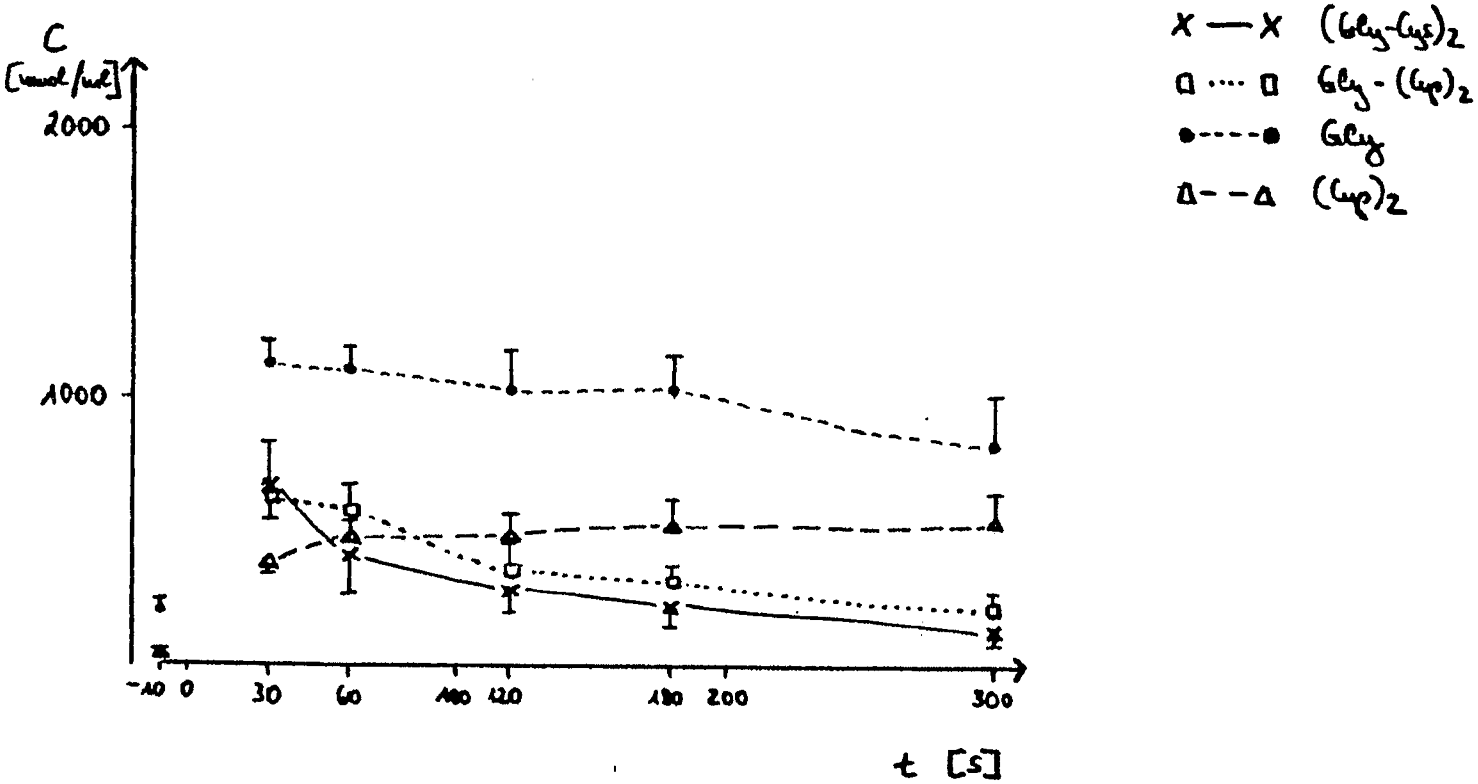


Abbildung 2: Plasma-Konzentrationen von $(Gly\text{-}Cys)_2$, $Gly\text{-}(Cys)_2$, Gly und $(Cys)_2$ nach Einmalinjektion von $(Gly\text{-}Cys)_2$ (20 µmol/100 g KG); Mittelwerte ± SEM, n = 6

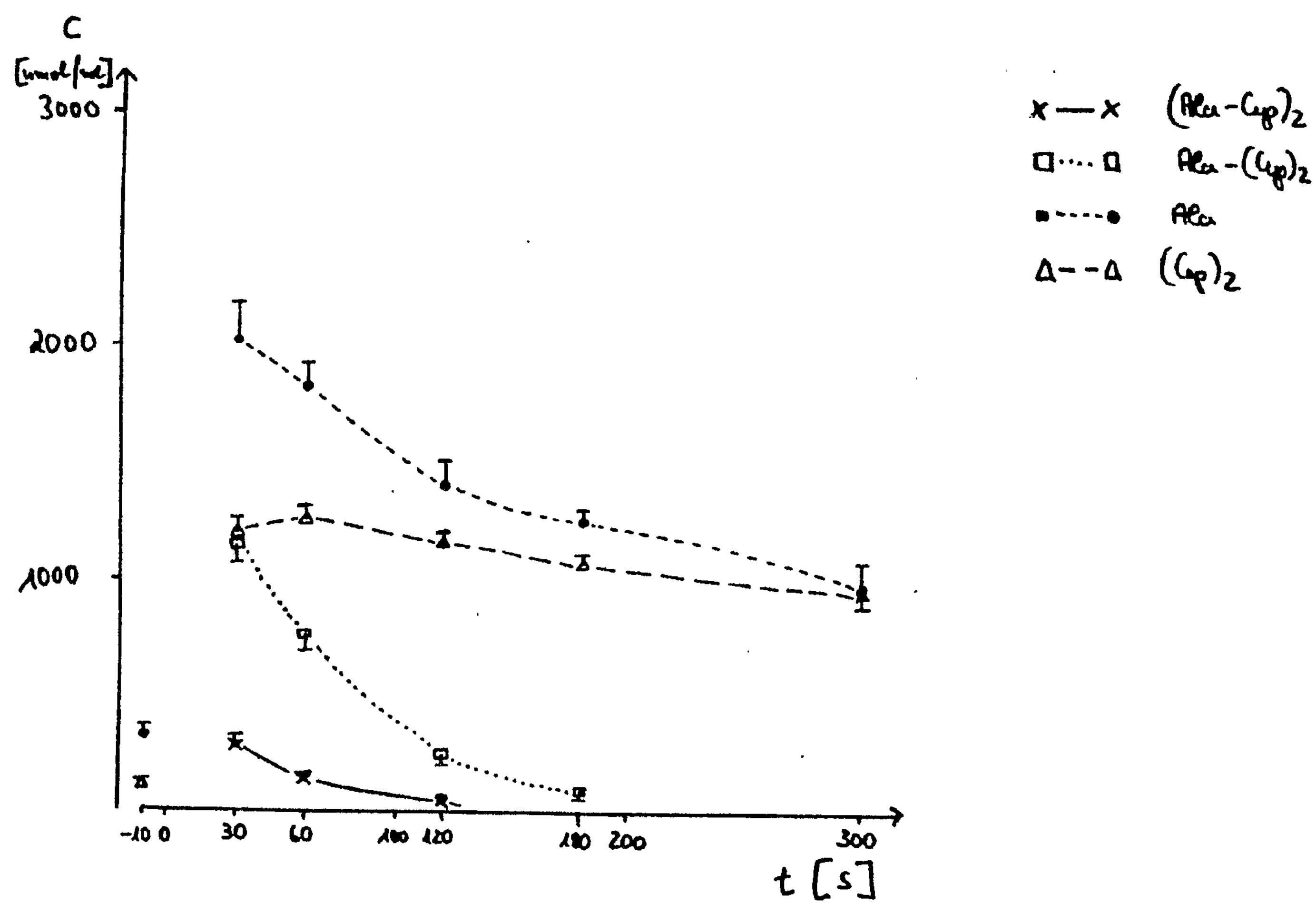


Abbildung 3: Plasma-Konzentrationen von $(Ala\text{-}Cys)_2$, $Ala\text{-}(Cys)_2$, Ala und $(Cys)_2$ nach Einmalinjektion von $(Ala\text{-}Cys)_2$ (20 µmol/100g KG); Mittelwerte ± SEM, n = 6